(19) Europäisches Patentamt
European Patent Office
Office européen des brevets

(11) Veröffentlichungsnummer: **0 233 151 B1**

# (12) EUROPÄISCHE PATENTSCHRIFT

(45) Veröffentlichungstag der Patentschrift: **13.11.91**

(51) Int. Cl.5: **C07D 209/48**, C07D 401/12, C07D 403/12, C07D 405/12, C07D 409/12, C07D 417/12, C07D 413/12, A01N 43/00

(21) Anmeldenummer: **87810071.8**

(22) Anmeldetag: **05.02.87**

Die Akte enthält technische Angaben, die nach dem Eingang der Anmeldung eingereicht wurden und die nicht in dieser Patentschrift enthalten sind.

## (54) Ester als Herbizide.

(30) Priorität: **11.02.86 CH 535/86**
**10.09.86 CH 3637/86**

(43) Veröffentlichungstag der Anmeldung:
**19.08.87 Patentblatt 87/34**

(45) Bekanntmachung des Hinweises auf die Patenterteilung:
**13.11.91 Patentblatt 91/46**

(84) Benannte Vertragsstaaten:
**AT BE CH DE ES FR GB GR IT LI LU NL SE**

(56) Entgegenhaltungen:
**EP-A- 0 216 243**
**GB-A- 2 071 100**
**GB-A- 2 150 929**
**JP-A-59 128 371**

(73) Patentinhaber: **CIBA-GEIGY AG**
**Klybeckstrasse 141**
**CH-4002 Basel(CH)**

(72) Erfinder: **Pissiotas, Georg, Dr.**
**Am Sonnenrain 71**
**W-7850 Lörrach(DE)**
Erfinder: **Moser, Hans, Dr.**
**Maispracherstrasse 12**
**CH-4312 Magden(CH)**
Erfinder: **Böhner, Beat, Dr.**
**Hügelweg 3**
**CH-4102 Binningen(CH)**

**EP 0 233 151 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Ester, speziell 5-(N-3,4,5,6-Tetrahydrophthalsäureimido)-benzoesäureester der Formel I mit herbizider und den Pflanzenwuchs regulierender Wirkung, sowie die Herstellung dieser neuen Ester. Ferner betrifft sie Mittel, welche die neuen Verbindungen enthalten sowie deren Verwendung zur selektiven Bekämpfung von Unkräutern oder zur Regulierung des Pflanzenwuchses.

Die neuen 5-(N-3,4,5,6-Tetrahydrophthalsäureimido)-benzoesäureester entsprechen der Formel I

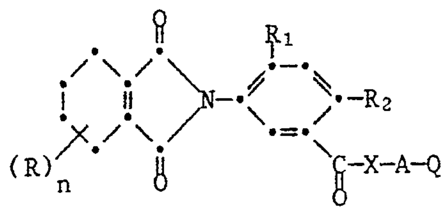

(I),

worin

- n Null, eins oder zwei,
- R $C_1$-$C_3$-Alkyl,
- $R_1$ Wasserstoff oder Halogen,
- $R_2$ Halogen,
- X Schwefel
- A eine geradkettige oder verzweigte $C_1$-$C_4$-Alkylenbrücke,
- Q den Rest $COOR_3$
- $R_3$ $C_1$-$C_{20}$-Alkyl, $C_3$-$C_6$-Alkenyl, $C_3$-$C_6$-Alkinyl oder $C_3$-$C_7$-Cycloalkyl unsubstituiert oder durch Halogen oder $C_1$-$C_4$-Alkoxy substituiert sein kann

bedeutet, mit Ausnahme der Verbindungen
2,4-Dichlor-5-(3,4,5,6-tetrahydrophthalimido)-thiobenzoesäure-S-methoxy-carbonylmethylester,
2,4-Dichlor-5-(3,4,5,6-tetrahydrophthalimido)-thiobenzoesäure-S-ethoxy-carbonyleth-1-ylester,
2,4-Difluor-5-(3,4,5,6-tetrahydrophthalimido)-thiobenzoesäure-S-ethoxy-carbonyleth-1-ylester,
2-Chlor-5-(3,4,5,6-tetrahydrophthalimido)-thiobenzoesäure-S-ethoxy-carbonyleth-1-ylester,
2-Brom-5-(3,4,5,6-tetrahydrophthalimido)-thiobenzoesäure-S-ethoxy-carbonyleth-1-ylester,
2-Fluor-5-(3,4,5,6-tetrahydrophthalimido)-thiobenzoesäure-S-ethoxy-carbonyleth-1-ylester.

Aus GB-A-2 071 100, GB-A-2 150 929 und JP-A-59-128 371 sind 5-(N-3,4,5,6-Tetrahydrophthalimido)-benzoesäureester und -thioester mit selektivherbizider Wirkung bekannt geworden. Sie sind von den erfindungsgemässen Verbindungen im Esterrest verschieden. In der nicht vorpublizierten EP-A 216 243 werden ferner herbizid wirkende 5-(N-3,4,5,6-Tetrahydrophthalimido)benzolsäure-thioester beschrieben, die im Phenylrest verschieden substituiert sind.

Die 5-(N-3,4,5,6-Tetrahydrophthalsäureimido)-benzoesäureester der Formel I zeichnen sich durch herbizide Wirkung aus. Je nach der Dosierung wirken sie als totale oder selektive Herbizide, und können zur Kontrolle von Unkräutern in Kulturen von Nutzpflanzen verwendet werden.

Diese Verbindungen besitzen ebenfalls eine den Pflanzenwuchs regulierende Wirkung und können zur Hemmung des Pflanzenwuchses verwendet werden.

Sie eignen sich z.B. zur Unterdrückung des Wachstums gewisser Pflanzen über das 2-Blattstadium hinaus (Solche Pflanzen sind beispielsweise Bodenbedecker-Leguminosen (Centrosema sp), welche in tropischen Kulturen zwischen die Reihen der Kulturpflanzen gesät werden, zwecks Erhaltung der Feuchtigkeit und Verhinderung der Bodenerosion) und zur Defoliattion und Dessiccation von Baumwoll- und Kartoffelkulturen (zwecks Arbeitserleichterung kurz vor deren Ernte).

Als besonders aktiv erwiesen sich diejenigen Verbindungen, in denen

- n Null, eins oder zwei,
- R $C_1$-$C_3$- Alkyl,
- $R_1$ Halogen,
- $R_2$ Halogen,
- X Schwefel,
- A eine geradkettige oder verzweigte $C_1$- $C_4$- Alkylenbrücke und
- Q eine $C_2$- $C_8$- Alkoxycarbonylgruppe

bedeutet.

insbesondere die Verbindungen
5-(N-3,4,5,6-Tetrahydrophthalsäureimido)-2-chlor-4-fluor-benzoesäure-(1-isopropoxycarbonyläth-1-yl)-thioester.
5-(N-3,4,5,6-Tetrahydrophthalsäureimido)-2-chlor-4-fluor-benzoesaure-(1-äthoxycarbonyläth-1-yl)-thioester,
5-(N-3,4,5,6-Tetrahydrophthalsäureimido)-2-chlor-4-fluor-benzoesäure-(decyloxycarbonylmethyl)-thioester,
5-(N-3,4,5,6-Tetrahydrophthalsäureimido)-2-chlor-4-fluor-benzoesäure-(cyclohexyloxycarbonylmethyl)-thioester,
5-(N-3,4,5,6-Tetrahydrophthalsäureimido)-2-chlor-4-fluor-benzoesäure(n-butoxycarbonlmethyl)-thioester,
5-(N-3,4,5,6-Tetrahydrophthalsäureimido)-2-chlor-4-fluor-benzoesäure-(hept-3-yloxycarbonylmethyl)-thioester,
5-(N-3,4,5,6-Tetrahydrophthalsäureimido)-2-chlor-4-fluor-benzoesäure-(methoxyethoxycarbonylmethyl)-thioester,
5-(N-3,4,5,6-Tetrahydrophthalsäureimido)-2-chlor-benzoesäure-(methoxycarbonylmethyl)-thioester,
5-(N-3,4,5,6-Tetrahydrophthalsäureimido)-2-chlor-benzoesäure-(methoxyäthoxycarbonylmethyl)-thioester,
5-(N-3,4,5,6-Tetrahydrophthalsäureimido)-2-chlor-benzoesäure-(n-butoxycarbonylmethyl)-thioester,
5-(N-3,4,5,6-Tetrahydrophthalsäureimido)-2-chlor-benzoesäure-(isopropoxycarbonylmethyl)-thioester,
5-(N-3,4,5,6-Tetrahydrophthalsäureimido)-2-chlor-benzoesäure-(cyclohexyloxycarbonylmethyl)-thioester,
5-(N-3,4,5,6-Tetrahydrophthalsäureimido)-2-chlor-benzoesäure-(isopropoxycarbonyläth-1-yl)-thioester,
5-(N-3,4,5,6-Tetrahydrophthalsäureimido)-2-chlor-4-fluor-benzoesäure-(methoxycarbonylmethyl)-thioester,
5-(N-3,4,5,6-Tetrahydrophthalsäureimido)-2-chlor-4-fluor-benzoesäure-(isopropoxycarbonylmethyl)-thioester,
5-(N-3,4,5,6-Tetrahydrophthalsäureimido)-2-chlor-4-fluor-benzoesäure(äthoxycarbonylmethyl)-thioester,
5-(N-3,4,5,6-Tetrahydrophthalsäureimido)-2-chlor-benzoesäure-(methoxycarbonyläth-1-yl)-thioester,
5-(N-3,4,5,6-Tetrahydrophthalsäureimido)-2-chlor-4-fluor-benzoesäure-(methoxycarbonyläth-1-yl)-thioester,
5-(N-3,4,5,6-Tetrahydrophthalsäureimido)-2-chlor-benzoesäure-(äthoxycarbonylmethyl)-thioester,
5-(N-3,4,5,6-Tetrahydrophthalsäureimido)-2-chlor-4-fluor-benzoesäure-(allyloxycarbonyläth-1-yl)-thioester,
5-(N-3,4,5,6-Tetrahydrophthalsäureimido)-2-chlor-4-fluor-benzoesäure-(propinyloxycarbonylmethyl)-thioester.

Die neuen 5-(N-3,4,5,6-Tetrahydrophthalsäureimido)-benzoesäureester der Formel I werden erfindungsgemäss hergestellt, indem man das Anhydrid einer 3,4,5,6-Tetrahydrophthalsäure der Formel II

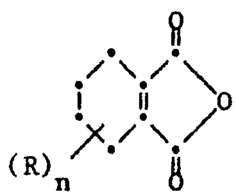

(II)

worin R und n die unter der Formel I gegebene Bedeutung haben, in einem inerten organischen Lösungs- oder Verdünnungsmittel, gegebenenfalls in Gegenwart einer geringen Menge eines wasserentziehenden Mittels, mit der äquimolaren Menge eines Derivates einer 3-Aminobenzoesäureester der Formel III umsetzt

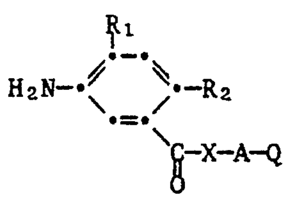

(III),

worin A, Q, $R_1$, $R_2$ und X die unter der Formel I gegebene Bedeutung haben.

Als Lösungs- oder Verdünnungsmittel eignen sich für diese Umsetzung höhersiedende Kohlenwasserstoffe, niedere Alkansäuren sowie deren Ester, höhersiedende Ketone und Aether. Als Beispiele seien Toluol, Xylol, Essigsäure, Essigsäureäthylester, Isopropyläther, Tetrahydrofuran, Methyläthylketon, sowie Dimethylformamid erwähnt.

Die Umsetzung findet bei einer Temperatur, die zwischen der Raumtemperatur und dem Siedepunkt des Reaktionsgemisches liegt, statt.

Zur Beschleunigung der Reaktion kann eine geringe Menge eines wasserentziehenden resp. wasserabsorbierenden Mittels zugegeben werden, wie beispielsweise Schwefelsäure oder einer organischen Sulfonsäure, eines Salzes, wie Natriumacetat eines Anhydrides, wie Phosphorpentoxid.

Das 3-Aminobenzoesäurederivat der Formel III kann z.B. entsprechend der folgenden Synthese hergestellt werden:

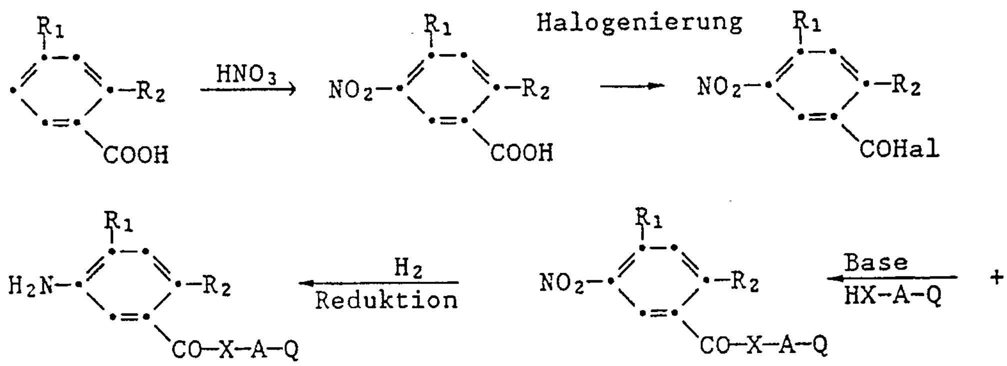


III

In diesen Formeln haben $R_1$, $R_2$, X, A und Q die unter der Formel I gegebene Bedeutung.

Eine im Phenylkern entsprechend substituierte Benzoesäure wird mit einem Salpetersäuregemisch nitriert und die entstandene 3-Nitrobenzoesäure anschliessend zum Säurehalogenid umgewandelt, z.B. mittels Phosphoroxychlorid oder -bromid, Thionylchlorid oder -bromid oder Sulfurylchlorid oder -bromid.

Das entstandene Säurehalogenid wird dann in Gegenwart der molaren Menge einer Base mit einem Thiol der Formel HS-A-Q umgesetzt, wobei A und Q die unter Formel I gegebene Bedeutung haben.

Das 3-Nitrobenzoesäurederivat kann aber auch direkt in Gegenwart einer Base mit einem Alkylhalogenid Hal-A-Q, worin A, X und Q die unter Formel I gegebene Bedeutung haben und Hal ein Halogenatom, vorzugsweise Chlor oder Brom bedeuten, kondensiert werden.

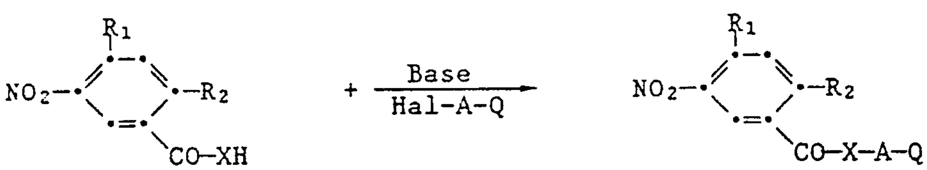


Der erhaltene 3-Nitrobenzoesäureester wird dann mit Wasserstoff z.B. in Gegenwart von Raney Nickel zum 3-Amino-benzoesäureester reduziert. Das Amin wird anschliessend mit einem 3,4,5,6-Tetrahydrophthalsäureanhydrid kondensiert, entsprechend dem erfindungsgemässen Verfahren.

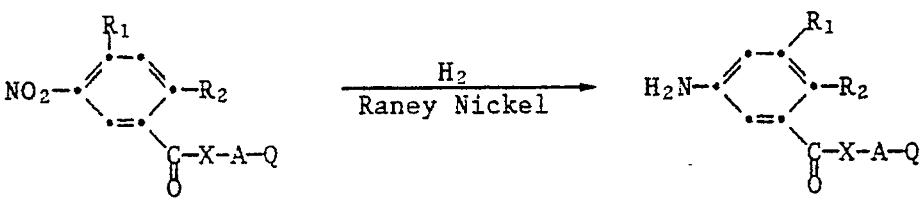


III

Gemäss einem weiteren erfindungsgemässen Verfahren erhält man die 5-(N-3,4,5,6-Tetrahydrophthalsäureimido)-benzoesäureester der Formel I Anspruch 1, indem man ein 5-(N-3,4,5,6-Tetrayhydrophthalsäureimido)-benzoesäurehalogenid der Formel V

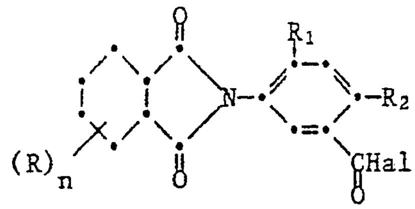

(V)

worin n, R, $R_1$ und $R_2$ die unter der Formel I gegebene Bedeutung haben und Hal ein Halogenatom, vorzugsweise Chlor oder Brom bedeutet, in einem inerten organischen Lösungs- oder Verdünnungsmittel in Gegenwart einer Base mit einem Thiol der Formel VI umsetzt

HX - A - Q (VI)

worin A, X und Q die unter der Formel I gegebene Bedeutung haben.

Als Lösungs- oder Verdünnungsmittel kommen auch für diese Umsetzung höhersiedende Kohlenwasserstoffe, niedere Ester von Alkansäuren, höhersiedende Ketone und Aether in Frage. Als Beispiel seien Hexan, Benzol, Toluol, Xylol, Essigsäureäthylester, Isopropyläther, Dioxan, Methyläthylketon erwähnt.

Die Umsetzung findet bei einer Temperatur statt, die zwischen 0 °C und dem Siedepunkt des Reaktionsgemisches liegt.

Als Basen kommen sekundäre und tertiäre Amine, Ammoniumbasen sowie die Hydroxide, Carbonate und Bicarbonate von Alkalimetallen in Frage. Als Beispiele seien Diäthylamin, Triäthylamin, Pyridin, Collidin, Natriumhydroxid, Natriumcarbonat oder -bicarbonat, Ammoniak genannt.

Das 5-(N-3,4,5,6-Tetrahydrophthalsäureimido)-benzoesäurehalogenid der Formel V wird durch Kondensation eines Phthalsäureanhydrides der Formel II mit einer entsprechend durch $R_1$ und $R_2$ substituierten 3-Aminobenzoesäure und anschliessender Halogenierung gemäss dem beschriebenen Verfahren hergestellt.

Ein weiteres Verfahren zur Herstellung der 5-(N-3,4,5,6-Tetrahydrophthalsäureimido)benzoesäureester der Formel I besteht darin, dass man eine 5-(N-3,4,5,6-Tetrahydrophthalsäureimido)-benzoesäure der Formel VII

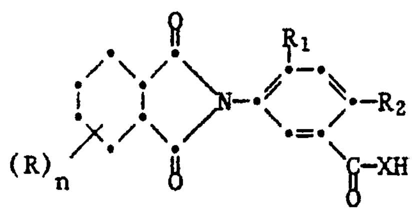

(VII)

worin n, R, $R_1$, $R_2$ und X die unter Formel I gegebene Bedeutung haben, in einem inerten organischen Lösungs- oder Verdünnungsmittel in Gegenwart der molaren Menge einer Base mit einem Alkylhalogenid der Formel VIII umsetzt

Hal - A - Q (III)

worin A und Q die im Anspruch 1 gegebene Bedeutung haben und Hal für ein Halogenatom, vorzugsweise Chlor oder Brom steht.

Als Lösungs- oder Verdünnungsmittel kommen die unter dem ersten oder zweiten Verfahren aufgezählten in Frage.

Die Umsetzung findet bei einer Temperatur statt, die zwischen 0 °C und dem Siedepunkt des Reaktionsgemisches liegt.

Als Basen können auch hier die oben erwähnten sekundären oder tertiären Amine, Ammoniumbasen, Hydroxide, Carbonate oder Bicarbonate von Alkalimetallen eingesetzt werden.

Die Reaktionsbedingungen sind bei allen beschriebenen Verfahren ähnlich.

Die Wirkstoff der Formel I werden in der Regel mit Aufwandmengen von 0,05 bis 4 kg/ha insbesondere 0,1 bis 1 kg/ha erfolgreich eingesetzt.

Bei geringeren Aufwandmengen zeichnen sich die Verbindungen der Formel I durch gute selektiv-wuchshemmende und selektiv-herbizide Eigenschaften aus, die sie ausgezeichnet zum Einsatz in Kulturen von Nutzpflanzen, insbesondere in Getreide, Baumwolle, Soja, Mais und Reis, befähigen. Es werden dabei teilweise auch Unkräuter geschädigt, welchen bisher nur mit Totalherbiziden beizukommen war.

Die Wirkungsart dieser Wirkstoffe ist unüblich. Viele sind translozierbar, d.h. sie werden von der Pflanze aufgenommen und an andere Stellen transportiert, wo sie dann zur Wirkung kommen. So gelingt es beispielsweise, durch Oberflächenbehandlung perennierende Unkräuter bis in die Wurzeln zu schädigen. Die neuen Verbindungen der Formel I wirken bereits bei - im Vergleich zu anderen Herbiziden und Wuchsregulatoren - sehr geringen Aufwandmengen.

Die Verbindungen der Formel I haben ausserdem starke pflanzenwuchshemmende, Eigenschaften. Es werden sowohl Monokotyledonen als auch Dikotyledonen in ihrem Wachstum beeinträchtigt.

So können z.B. die in der Landwirtschaft in tropischen Gegenden häufig als "cover crops" (Bodenbedecker) angepflanzten Leguminosen durch die Verbindungen der Formel I in ihrem Wachstum selektiv gehemmt werden, so dass zwar die Bodenerosion zwischen den Kulturpflanzen verhindert wird, die "cover crops" jedoch nicht zur Konkurrenz für die Kultur werden können.

Eine Hemmung des vegetativen Wachstums erlaubt bei vielen Kulturpflanzen eine dichtere Anpflanzung der Kultur, so dass ein Mehrertrag, bezogen auf die Bodenfläche, erzielt werden kann.

Ein weiterer Mechanismus der Ertragssteigerung mit Wachstumshemmern beruht darauf, dass die Nährstoffe in stärkerem Masse der Blüten-und Fruchtbildung zugute kommen, während das vegetative Wachstum eingeschränkt wird.

Die Wirkstoffe der Formel I können ferner zur Defoliation und Dessiccation von Baumwoll- und Kartoffelkulturen eingesetzt werden. Durch Behandeln der Kulturen im Zeitpunkt der Reife wird die Ernte der Baumwollkapseln oder der Knollen stark erleichtert, wenn die Blätter und Stauden abgefallen und/oder zur Dürre getrocknet sind.

Bei grösseren Aufwandmengen werden alle getesteten Pflanzen in ihrer Entwicklung so geschädigt, dass sie absterben.

Die Erfindung betrifft auch herbizide und pflanzenwachstumsregulierende Mittel, welche einen neuen Wirkstoff der Formel I enthalten, sowie Verfahren zur pre- und post-emergenten Unkrautbekämpfung und zur Hemmung des Pflanzenwuchses von monokotylen und dikotylen Pflanzen, insbesondere Gräsern, tropischen Bodenbedeckern und Tabakgeiztrieben.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dicotylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder äthyläther, Ketone wie Cyclohexanon, stark polare Lösungsmittel wie N-Methyl-2-pyrrolidon,Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäuremethyl-taurinsalzezu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 10 Glykoläthergruppen und 8 bis 20 Kohlensotffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffdatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthanol Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgenden Publikationen beschrieben:

"Mc Cutcheon's Detergents and Emulsifiers Annual"
MC Publishing Corp., Ridgewood, New Jersey, 1979.
Dr. Helmut Stache "Tensid Taschenbuch"
Carl Hanser Verlag, München/Wien 1981.

Die pestiziden Zubereitungen enthalten in der Regel 0,1 bis 95 %, insbesondere 0,1 bis 80 %, Wirkstoff der Formel I, 1 bis 99,9 % eines festen oder flüssigen Zusatzstoffes und 0 bis 25 %, insbesondere 0,1 bis 25 % eines Tensides.

Insbesondere setzen sich bevorzugte Formulierungen folgendermassen zusammen: (% = Gewichtsprozent)

Emulgierbare Konzentrate:

Wirkstoff der Formel I: 1 bis 20 % bevorzugt 5 bis 10 %
oberflächenaktives Mittel: 5 bis 30 %,vorzugsweise 10 bis 20 %
flüssiges Trägermittel: 50 bis 94 %,vorzugsweise 70 bis 85 %.

Stäube:

Wirkstoff der Formel I: 0,1 bis 10 %, vorzugsweise 0,1 bis 1 %

festes Trägermittel: 99,9 bis 90 %, vorzugsweise 99,9 bis 99 %.

Suspensions-Konzentrate:

Wirkstoff der Formel I: 5 bis 75 %, vorzugsweise 10 bis 50 %
Wasser: 94 bis 25 %, vorzugsweise 90 bis 30 %
oberflächenaktives Mittel: 1 bis 40 %, vorzugsweise 2 bis 30 %.

Benetzbare Pulver:

Wirkstoff der Formel I: 0,5 bis 90 %, vorzugsweise 1 bis 80 %
oberflächenaktives Mittel:0,5 bis 20 %, vorzugsweise 1 bis 15 %
festes Trägermittel: 5 bis 95 %, vorzugsweise 15 bis 90 %.

Granulate:

Wirkstoff der Formel I: 0,5 bis 30 %, vorzugsweise 3 bis 15 %
festes Trägermittel: 99,5 bis 70 %, vorzugsweise 97 bis 85 %.

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,005 bis 5 kg As/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Das folgende Beispiel veranschaulicht die Herstellung eines 5-(3,4,5,6-Tetrahydrophthalsäureimido)-2-chlor-4-fluor-benzoesäureesters. In entsprechender Weise werden die in der anschliessenden Tabelle aufgeführten erfindungsgemässen Wirkstoffe hergestellt.

Beispiel 1

Herstellung von Chlor-4-fluor-5-(N-3,4,5,6-tetrahydrophthalsäureimido)-benzoesäure-pyridin-2-yl-äthylester

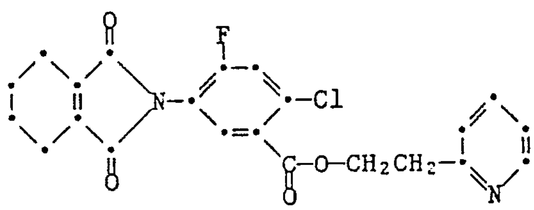

Zu einem Gemisch von 3,4 g 2-Chlor-4-fluor-5-(N-3,4,5,6-tetrahydrophthalsäureimido)-benzoesäurechlorid, 1,2 ml 2-(2-Hydroxyäthyl)-pyridin und 30 ml Toluol tropft man langsam, unter Rühren 1,2 ml Triäthylamin. Nach dreistündigem Rühren bei Raumtemperatur filtriert man das Reaktionsgemisch und dampft das Filtrat im Vakuum ein. Es verbleiben 3,4 g Titelprodukt als helles Oel mit Brechungsindex $n_D^{23}$ 1.5581.

In analoger Weise zu diesem Beispiel werden die Verbindungen der Tabelle 1 hergestellt:

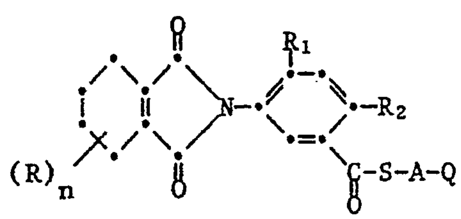

Tabelle 1

| No. | $(R)_n$ | $R_1$ | $R_2$ | A | Q | phys. Daten |
|---|---|---|---|---|---|---|
| 1 | - | F | Cl | $CH(CH_3)$ | $COOCH(CH_3)_2$ | $n_D^{23}$ 1.5421 |
| 2 | - | F | Cl | $CH(CH_3)$ | $COOC_2H_5$ | $n_D^{23}$ 1.5357 |
| 3 | - | F | Cl | $CH_2$ | $COO(CH_2)_{17}CH_3$ | wachsartig |
| 4 | - | F | Cl | $CH_2$ | $COO(CH_2)_9CH_3$ | $n_D^{24}$ 1.5254 |

| No. | $(R)_n$ | $R_1$ | $R_2$ | A | Q | phys. Daten |
|---|---|---|---|---|---|---|
| 5 | - | F | Cl | $CH_2$ | COOCyclohexyl | $n_D^{24}$ 1.5519 |
| 6 | - | F | Cl | $CH_2$ | $COO(CH_2)_{15}CH_3$ | $n_D^{24}$ 1.5207 |
| 7 | - | F | Cl | $CH_2$ | $COO(CH_2)_{11}CH_3$ | $n_D^{24}$ 1.5274 |
| 8 | - | F | Cl | $CH_2$ | $COOC_4H_9n$ | $n_D^{24}$ 1.5525 |
| 9 | - | F | Cl | $CH_2$ | $COOCH(C_2H_5)C_4H_9n$ | $n_D^{24}$ 1.5388 |
| 10 | - | F | Cl | $CH_2$ | $COOC_2H_4OCH_3$ | $n_D^{24}$ 1.5542 |
| 11 | - | H | Cl | $CH_2$ | $COOCH_3$ | $n_D^{24}$ 1.5832 |
| 12 | - | H | Cl | $CH_2$ | $COOC_2H_4OCH_3$ | $n_D^{24}$ 1.5662 |
| 13 | - | H | Cl | $CH_2$ | $COOCH(C_2H_5)C_4H_9n$ | $n_D^{24}$ 1.5431 |
| 14 | - | H | Cl | $CH_2$ | $COOC_4H_9n$ | $n_D^{24}$ 1.5639 |
| 15 | - | H | Cl | $CH_2$ | $COO(CH_2)_9CH_3$ | $n_D^{24}$ 1.5425 |
| 16 | - | H | Cl | $CH_2$ | $COO(CH_2)_{11}CH_3$ | $n_D^{24}$ 1.5342 |
| 17 | - | H | Cl | $CH_2$ | $COOCH(CH_3)_2$ | $n_D^{24}$ 1.5659 |
| 18 | - | H | Cl | $CH_2$ | $COO(CH_2)_{15}CH_3$ | $n_D^{24}$ 1.5292 |
| 19 | - | H | Cl | $CH_2$ | $COO(CH_2)_{17}CH_3$ | $n_D^{24}$ 1.5415 |
| 20 | - | H | Cl | $CH_2$ | COOcyclohexyl | $n_D^{24}$ 1.5545 |
| 21 | - | H | Cl | $CH(CH_3)$ | $COOCH(CH_3)_2$ | $n_D^{24}$ 1.5532 |
| 22 | - | F | Cl | $CH_2$ | $COOCH_3$ | $n_D^{23}$ 1.5532 |
| 23 | - | F | Cl | $CH_2$ | $COOCH(CH_3)_2$ | $n_D^{20}$ 1.5623 |
| 24 | - | F | Cl | $CH_2$ | $COOC_2H_5$ | $n_D^{22}$ 1.5638 |
| 25 | - | H | Cl | $CH(CH_3)$ | $COOCH_3$ | $n_D^{21}$ 1.5703 |
| 26 | - | F | Cl | $CH(CH_3)$ | $COOCH_3$ | $n_D^{22}$ 1.5472 |
| 27 | - | H | Cl | $CH_2$ | $COOC_2H_5$ | $n_D^{25}$ 1.5720 |
| 28 | - | F | Cl | $CH_2$ | $COOCH_2CH{=}CH_2$ | |
| 29 | - | F | Cl | $CH(CH_3)$ | $COOCH_2CH{=}CH_2$ | |

| No. | $(R)_n$ | $R_1$ | $R_2$ | A | Q | phys. Daten |
|---|---|---|---|---|---|---|
| 30 | - | H | Cl | $CH_2$ | $COOCH_2CH{=}CH_2$ | |
| 31 | - | H | Cl | $CH(CH_3)$ | $COOCH_2CH{=}CH_2$ | |
| 32 | - | F | Cl | $CH_2$ | $COOCH_2C{\equiv}CH$ | |
| 33 | - | F | Cl | $CH(CH_3)$ | $COOCH_2C{\equiv}CH$ | |
| 34 | - | H | Cl | $CH_2$ | $COOCH_2C{\equiv}CH$ | |
| 35 | - | H | Cl | $CH(CH_3)$ | $COOCH_2C{\equiv}CH$ | |
| 36 | - | H | Cl | $CH(CH_3)$ | $COOCH(CH_3)_2$ | $n_D^{24}$ 1.5532 |
| 37 | - | F | Br | $CH(CH_3)$ | $COOC_2H_5$ | $n_D^{36}$ 1.5571 |
| 38 | - | F | Br | $CH_2$ | $COOCH_3$ | $n_D^{25}$ 1.5814 |
| 39 | - | F | Br | $CH_2$ | $COOC_2H_5$ | $n_D^{25}$ 1.5622 |
| 40 | - | F | Br | $CH_2$ | $COOC_3H_7$i-iso | $n_D^{25}$ 1.5611 |
| 41 | - | F | Br | $CH(CH_3)$ | $COOCH_3$ | $n_D^{25}$ 1.5708 |
| 42 | - | F | Br | $CH(CH_3)$ | $COOC_3H_7$i-iso | $n_D^{25}$ 1.5623 |
| 43 | - | F | Br | $CH_2$ | $COOC_2H_4OCH_3$ | $n_D^{23}$ 1.5750 |
| 44 | - | F | Br | $CH_2$ | $COOC_4H_9$n | $n_D^{21}$ 1.5667 |

Formulierungsbeispiele:

Beispiel 2: Formulierungsbeispiele für Wirkstoffe der Formel I (% = Gewichtsprozent)

| a) Spritzpulver | a) | b) | c) |
|---|---|---|---|
| Wirkstoff gemäss Tabelle 1 | 20 % | 60 % | 0,5 % |
| Na-Ligninsulfonat | 5 % | 5 % | 5 % |
| Na-Laurylsulfat | 3 % | - | - |
| Na-Diisobutylnaphthalinsulfonat | - | 6 % | 6 % |
| Octylphenolpolyäthylenglykoläther (7-8 Mol AeO) | - | 2 % | 2 % |
| Hochdisperse Kieselsäure | 5 % | 27 % | 27 % |
| Kaolin | 61 % | - | - |
| Natriumchlorid | - | - | 59,5 % |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| b) Emulsions-Konzentrat | a) | b) |
|---|---|---|
| Wirkstoff gemäss Tabelle 1 | 10 % | 1 % |
| Octylphenolpolyäthylenglykoläther (4-5 Mol AeO) | 3 % | 3 % |
| Ca-Dodecylbenzolsulfonat | 3 % | 3 % |
| Ricinusölpolyglykoläther (36 Mol AeO) | 4 % | 4 % |
| Cyclohexanon | 30 % | 10 % |
| Xylolgemisch | 50 % | 79 % |

Aus diesem Konzentrat können durch Verdünnen mit Wasser Emulsionen jeder gewünschten Konzentration hergestellt werden.

| c) Stäubemittel | a) | b) |
|---|---|---|
| Wirkstoff gemäss Tabelle 1 | 0,1 % | 1 % |
| Talkum | 99,9 % | - |
| Kaolin | - | 99 % |

Man erhält anwendungsfertige Stäubemittel, indem der Wirkstoff mit dem Träger vermischt und auf einer geeigneten Mühle vermahlen wird.

| d) Extruder Granulat | a) | b) |
|---|---|---|
| Wirkstoff gemäss Tabelle 1 | 10 % | 1 % |
| Na-Ligninsulfonat | 2 % | 2 % |
| Carboxymethylcellulose | 1 % | 1 % |
| Kaolin | 87 % | 96 % |

Der Wirkstoff wird mit den Zusatzstoffen vermischt, vermahlen und mit Wasser angefeuchtet. Dieses Gemisch wird extrudiert und anschliessend im Luftstrom getrocknet.

| e) Umhüllungs-Granulat | |
|---|---|
| Wirkstoff gemäss Tabelle 1 | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| f) Suspensions-Konzentrat | a) | b) |
|---|---|---|
| Wirkstoff gemäss Tabelle 1 | 40 % | 5 % |
| Aethylenglykol | 10 % | 10 % |
| Nonylphenolpolyäthylenglykoläther (15 Mol AeO) | 6 % | 1 % |
| Na-Ligninsulfonat | 10 % | 5 % |
| Carboxymethylcellulose | 1 % | 1 % |
| 31 %ige wässrige Formaldehyd-Lösung | 0,2 % | 0,2 % |
| Silikonöl in Form einer 75%igen wässrigen Emulsion | 0,8 % | 0,8 % |
| Wasser | 32 % | 77 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

| g) Salzlösung | |
|---|---|
| Wirkstoff gemäss Tabelle 1 | 5 % |
| Isopropylamin | 1 % |
| Octylphenolpolyäthylenglykoläther (78 Mol AeO) | 3 % |
| Wasser | 91 % |

Biologische Beispiele:

Beispiel 3: Pre-emergente Herbizid-Wirkung

Im Gewächshaus wird unmittelbar nach der Einsaat der Versuchspflanzen in Saatschalen die Erdoberfläche mit einer wässrigen Dispersion der Wirkstoffe, erhalten aus einem 25 %igen Emulsions-konzentrat behandelt. Es werden Konzentrationen von 4 kg Wirksubstanz/Hektar angewendet. Die Saatschalen werden im Gewächshaus bei 22-25° C und 50-70 % relativer Luftfeuchtigkeit gehalten und der Versuch nach 3 Wochen ausgewertet. Der Zustand der Pflanzen wurde gemäss folgender Notenskala boniliert

| | |
|---|---|
| 1 | Pflanze abgestorben oder nicht zum Keimen gekommen |
| 2-4 | starke, graduell abnehmende Schädigung |
| 5 | mittlere Schädigung |
| 6-8 | leichtere Schadstufen |
| 9 | Pflanze wächst normal, wie unbehandelte Kontrollpflanzen. |

Beispiel 4: Post-emergente Herbizid-Wirkung (Kontaktherbizid)

Eine Anzahl Unkräuter, sowohl monokotyle wie dikotyle, wurden nach dem Auflaufen (im 4- bis 6-Blattstadium) mit einer wässrigen Wirkstoffdispersion in einer Dosierung von 4 kg Wirksubstanz pro Hektar auf die Pflanzen gespritzt und diese bei 24°-26° C und 45-60 % relativer Luftfeuchtigkeit gehalten. 15 Tage nach der Behandlung wird der Versuch nach der obigen Notenskala ausgewertet.

Beispiel 5: Herbizidwirkung vor dem Auflaufen der Pflanzen

Kunststofftöpfe werden mit expandiertem Vermiculit (Dichte: 0,135 g/cm$^3$, Wasserabsorptionsvermögen: 0,565 1/1) gefüllt. Nach dem Sättigen des nicht adsorptiven Vermiculits mit einer wässrigen Wirkstoffemulsion in deinonisiertem Wasser, die die Wirkstoffe in einer Konzentration von 70,8 ppm enthält, werden Samen der folgenden Pflanzen auf die Oberfläche gesät. Nasturtium offcinalis, Agrostis tenuis, Stellaria media und Digitaria sanguinalis. Die Versuchsgefässe werden anschliessend in einer Klimakammer bei 20° C, einer Beleuchtung von ca. 20 kLux und einer relativen Luftfeuchtigkeit von 70 % gehalten. Während der Keimphase von 4 bis 6 Tagen werden die Töpfe zur Erhöhung der örtlichen Luftfeuchtigkeit mit lichtdurchlässigem Material abgedeckt und mit deinoisiertem Wasser begossen. Nach dem 5. Tag wird dem Giesswasser 0,5 % eines handelsüblichen Flüssigdüngers (®Greenzit) zugesetzt. 12 Tage nach der Aussaat wird der Versuch ausgewertet und die Wirkung auf die Versuchspflanzen bewertet.

Beispiel 6 Herbizidwirkung für Wasserreis (paddy)

Die Wasserunkräuter Echinochloa crus galli und Monocharia vag. werden in Plastikbechern (60 cm$^2$ Oberfläche, 500 ml Volumen) ausgesät. Nach der Saat wird bis zur Erdoberfläche mit Wasser aufgefüllt. 3 Tage nach der Saat wird der Wasserspiegel bis leicht über die Erdoberfläche erhöht (3-5 mm). Die Applikation erfolgt 3 Tage nach der Saat mit einer wässrigen Emulsion der Prüfsubstanzen durch eine Spritzung auf die Gefässe. Die daher verwendete Dosis entspricht einer Wirkstoffmenge von 0,5 kg Wirkstoff pro Hektar (Spritzbrühmenge = 550 1/ha. Die Pflanzenbecher werden dann im Gewächshaus unter optimalen Wachstumsbedingungen für die Reisunkräuter aufgestellt, d.h. bei 25°-30° C und hoher Luftfeuchtigkeit. Die Auswertung der Versuche findet 3 Wochen nach Applikation statt. Der Zustand der Pflanzen wird nach der oben gegebenen Notenskala verwertet.

Beispiel 7: Desiccations- und Defoliationswirkung

In einem Gewächshaus wurden Baumwollpflanzen der Sorte Deltapine in Tontöpfen angezogen. Nach

abgeschlossener Kapselbildung wurden sie mit wässerigen Zubereitungen des Wirkstoffes in einer Aufwandmenge, die 1,2, 0,6 und 0,3 kg/ha im Feld entspräche, gespritzt. Unbehandelte Pflanzen wurden als Kontrolle belassen. Die Auswertung des Versuches erfolgte 3, 7 und 14 Tage nach Applikation der Wirksubstanz durch Bestimmen des Grades an Defoliation (% abgefallene Blätter) und an Dessikation (% Austrocknung der an der Pflanze verbleibenden Blätter).

Beispiel 8: Wuchshemmung bei tropischen Bodenbedecker-Leguminosen (cover crops).

Die Versuchspflanzen (centrosema plumieri und centrosema pubescens) werden bis zum ausgewachsenen Stadium herangezogen und bis auf eine Höhe von 60 cm zurückgeschnitten. Nach 7 Tagen wird der Wirkstoff als wässrige Emulsion gespritzt. Die Versuchspflanzen werden bei 70 % relativer Luftfeuchtigkeit und 6000 lux Kunstlicht, pro Tag 14 Stunden, bei Temperaturen von 27° bei Tag und 21°C bei Nacht gehalten. 4 Wochen nach der Applikation wird der Versuch ausgewertet. Es werden dabei der Neuzuwachs im Vergleich zur Kontrolle abgeschätzt und gewogen und die Phytotoxizität bewertet.

## Patentansprüche

**1.** 5-(N-3,4,5,6-Tetrahydrophthalsäureimido)-benzoesäureester der Formel I

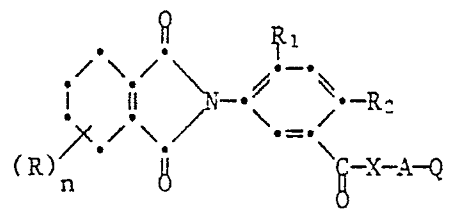

(I),

worin

- n Null, eins oder zwei,
- R $C_1$-$C_3$-Alkyl,
- $R_1$ Wasserstoff oder Halogen,
- $R_2$ Halogen,
- X Schwefel
- A eine geradkettige oder verzweigte $C_1$-$C_4$-Alkylenbrücke,
- $R_3$ $c_1$-$c_{20}$-Alkyl, $C_3$-$C_6$-Alkenyl, $C_3$-$C_4$-Alkinyl oder $C_3$-$C_7$-Cycloalkyl, welches unsubstituiert oder durch Halogen oder $C_1$-$C_4$-Alkoxy substituiert sein kann

bedeutet;
mit Ausnahme der Verbindungen
2,4-Dichlor-5-(3,4,5,6-tetrahydrophthalimido)-thiobenzoesäure-S-methoxycarbonylmethylester,
2,4-Dichlor-5-(3,4,5,6-tetrahydrophthalimido)-thiobenzoesäure-S-ethoxycarbonyleth-1-ylester,
2,4-Difluor-5-(3,4,5,6-tetrahydrophthalimido)-thiobenzoesäure-S-ethoxycarbonyleth-1-ylester,
2-Chlor-5-(3,4,5,6-tetrahydrophthalimido)-thiobenzoesäure-S-ethoxycarbonyleth-1-ylester,
2-Brom-5-(3,4,5,6-tetrahydrophthalimido)-thiobenzoesäure-S-ethoxycarbonyleth-1-ylester,
2-Fluor-5-(3,4,5,6-tetrahydrophthalimido)-thiobenzoesäure-S-ethoxycarbonyleth-1-ylester.

**2.** 5-(N-3,4,5,6-Tetrahydrobenzoesäureimido)-benzoesäureester gemäss Anspruch 1, in deren

- n Null, eins oder zwei,
- R $C_1$-$C_3$-Alkyl,
- $R_1$ Wasserstoff oder Halogen,
- $R_2$ Halogen,
- X Schwefel,
- A eine geradkettige oder verzweigte $C_1$-$C_4$-Alkylenbrücke und
- Q eine $C_2$-$C_8$-Alkoxycarbonylgruppe

bedeutet.

**3.** 5-(N-3, 4, 5, 6-Tetrahydrophthalsäureimido)-2-chlor-4-fluor-benzoesäure-(1-isopropoxycarbonyläth-1-yl)-thioester, gemäss Anspruch 1.

4. 5-(N-3, 4, 5, 6-Tetrahydrophthalsäureimido)-2-chlor-4-fluor-benzoesäure-(1-äthoxycarbonyläth-1-yl)-thioester, gemäss Anspruch 1.

5. 5-(N-3, 4, 5, 6-Tetrahydrophthalsäureimido)-2-chlor-4-fluor-benzoesäure-(decyloxycarbonylmethyl)-thioester, gemäss Anspruch 1.

6. 5-(N-3, 4, 5, 6-Tetrahydrophthalsäureimido)-2-chlor-4-fluor-benzoesäure-(cyclohexyloxycarbonylmethyl)-thioester, gemäss Anspruch 1.

7. 5-(N-3, 4, 5, 6-Tetrahydrophthalsäureimido)-2-chlor-4-fluor-benzoesäure-(n-butoxycarbonylmethyl)-thioester, gemäss Anspruch 1.

8. 5-(N-3, 4, 5, 6-Tetrahydrophthalsäureimido)-2-chlor-4-fluor-benzoesäure-(hept-3-yloxycarbonylmethy)-thioester, gemäss Anspruch 1.

9. 5-(N-3,4,5,6-Tetrahydrophthalsäureimido)-2-fluor-4-fluor-benzoesäure-(methoxyethoxycarbonylmethyl)-thioester, gemäss Anspruch 1.

10. 5-(3,4,5,6-Tetrahydrophthalsäureimido)-2-chlor-benzoesäure-(methoxycarbonylmethyl)-thioester, gemäss Anspruch 1.

11. 5-(N-3,4,5,6-Tetrahydrophthalsäureimido)-2-chlor-benzoesäure-(methoxyäthoxycarbonylmethyl)-thioester, gemäss Anspruch 1.

12. 5-(N-3,4,5,6-Terahydrophthalsäureimido)-2-chlor-benzoesäure-(n-butoxycarbonylmethyl)-thioester, gemäss Anspruch 1.

13. 5-(N-3,4,5,6-Tetrahydrophthalsäureimido)-2-chlor-benzoesäure-(isopropoxycarbonylmethyl)-thioester, gemäss Anspruch 1.

14. 5-(N-3,4,5,6-Tetrahydrophthalsäureimido)-2-chlor-benzoesäure-(cyclohexyloxycarbonylmethl)-thioester, gemäss Anspruch 1.

15. 5-(N-3,4,5,6-Tetrahydrophthalsäureimido)-2-chlor-henzoesäure-(isopropoxycarbonyläth-1-yl)-thioester, gemäss Anspruch 1.

16. 5(N-3,4,5,6-Tetrahydrophthalsäureimido)-2-chlor-4-fluor-benzoesäure-(methoxycarbonylmethyl)-thioester, gemäss Anspruch 1.

17. 5-(N-3,4,5,6-Tetrahydrophthalsäureimido)-2-chlor-4-fluor-benzoesäure-(isopropoxycarbonylmethyl)-thioester, gemäss Anspruch 1.

18. 5-(N-3,4,5,6-Tetrahydrophthalsäureimido)-2-chlor-4-fluor-henzoesäure-(äthoxycarbonylmethyl)-thioester, gemäss Anspruch 1.

19. 5-(N-3,4,5,6-Tetrahydrophthalsäureimido)-2-chlor-benzoesäure-(methoxycarbonyläth-1-yl)-thioester, gemäss Anspruch 1.

20. 5-(N-3,4,5,6-Terahydrophthalsäureimido)-2-chlor-4-benzoesäure-(methoxycarbonyläth-1-yl)-thioester, gemäss Anspruch 1.

21. 5-(N-3,4,5,6-Tetrahydrophthalsäureimido)-2-chlor-benzoesäure-(äthoxycarbonylmethyl)-thioester, gemäss Anspruch 1.

22. 5-(N-3,4,5,6-Tetrahydrothalsäureimido)-2-chlor-4-fluor-benzoe säure(allyloxycarbonyläth-1-yl)-thioester, gemäss Anspruch 1.

23. 5-(N-3,4,5,6-Tetrahydrophthalsäureimido)-2-chlor-4-fluor-benzoesäure-(propinyloxycarbonylmethyl)-thioester, gemäss Anspruch 1.

24. Verfahren zur Herstellung der 5-(N-3,4,5,6-Tetrahydrophthal säureimido)-benzoesäureester der Formel I, gemäss Anspruch 1.
dadurch gekennzeichnet, dass man das Anhydrid einer 3,4,5,6-Tetrahydrophthalsäure der Formel II

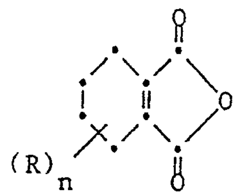

(II)

worin R und n die unter der Formel I gegebene Bedeutung haben, in einem inerten organischen Lösungs- oder Verdünnungsmittel, gegebenenfalls in Gegenwart einer geringen Menge eines wasserentziehenden Mittels, mit der äquimolaren Menge einer 3-Aminobenzoylverbindung der Formel III umsetzt,

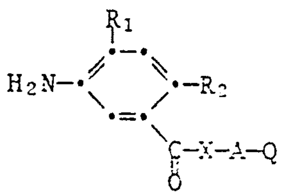

(III)

worin A, $R_1$, $R_2$, X und Q die im Anspruch 1 gegebene Bedeutung haben.

25. Verfahren zur Herstellung der 5-(N-3,4,5,6-Tetraphthalsäureimido)-benzoesäureester der Formel I, Anspruch 1, dadurch gekennzeichnet, dass man ein 5-(N-3,4,5,6-Tetrayhydrophthalsäureimido)-benzoesäurehalogenidder Formel V

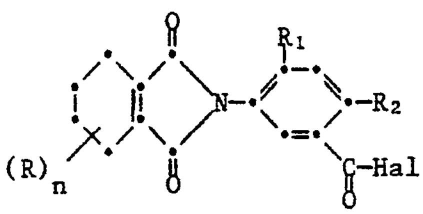

(V)

worin n, R, $R_1$ und $R_2$ die unter der Formel I gegebene Bedeutung haben und Hal ein Halogenatom, vorzugsweise Chlor oder Brom bedeutet, in einem inerten organischen Lösungs- oder Verdünnungsmittel in Gegenwart einer Base mit einem Thiol der Formel VI umsetzt

HX - A - Q (VI)

worin A, X und Q die unter der Formel I gegebene Bedeutung haben.

26. Verfahren zur Herstellung der 5-(N-3,4,5,6-Tetrahydrophthalsäureimido)benzoesäureester der Formel I, Anspruch 1, dadurch gekennzeichnet, dass man eine 5-(N-3,4,5,6-Tetrahydrophthalsäureimido)-benzoesäure der Formel VII

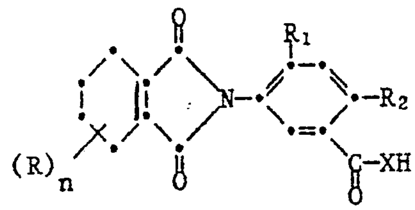

(VII)

worin n, R, $R_1$, $R_2$ und X die unter Formel I gegebene Bedeutung haben, in einem inerten organischen Lösungs- oder Verdünnungsmittel in Gegenwart der molaren Menge einer Base mit einem Alkylhalogenid der Formel VIII umsetzt,

Hal - A - Q (VIII)

worin A und Q die im Anspruch 1 gegebene Bedeutung haben und Hal für ein Halogenatom, vorzugsweise Chlor oder Brom steht.

27. Ein herbizides und Pflanzenwachstum regulierendes Mittel, dadurch gekennzeichnet, dass es neben Träger- und/oder anderen Zuschlagstoffen als Wirkstoff ein 5-(N-3,4,5,6-Tetrahydrophthalsäureimido)-benzoesäureester der Formel I, Anspruch 1 enthält.

28. Die Verwendung eines Wirkstoffes gemäss Anspruch 1, oder einen solchen Wirkstoff enthaltendes Mittel zur Bekämpfung unerwünschten Pflanzenwachstums.

29. Die Verwendung eines Wirkstoffes gemäss Anspruch 1 oder einen solchen Wirkstoff enthaltendes Mittel zur Hemmung des Pflanzenwachstums.

30. Die Verwendung eines Wirkstoffes gemäss Anspruch 1, oder einen solchen Wirkstoff enthaltendes Mittel zur Beeinflussung des Pflanzenwachstums zum Zweck der Ertragssteigerung.

31. Verfahren zur selektiven pre- oder postemergenten Bekämpfung von Unkräutern in Nutzpflanzenkulturen, dadurch gekennzeichnet, dass man die Nutzpflanzenkulturen oder deren Anbaufläche mit einer wirksamen Menge eines Wirkstoffes der Formel I, Anspruch 1 oder eines eine solche Verbindung enthaltenden Mittels behandelt.

32. Verfahren zur Unterdrückung des Pflanzenwachstums über das 2-Blattstadium hinaus, dadurch gekennzeichnet, dass man die Pflanzen während ihres Wachstums mit einer wirksamen Menge eines Wirkstoffes der Formel I Anspruch 1 oder eines einen solchen Wirkstoff enthaltendes Mittel behandelt.

33. Verfahren zur Defoliation und Dessiccation von Baumwoll- und Kartoffelkulturen zum Erleichtern der Ernte, dadurch gekennzeichnet, dass man die Kultur kurz vor der Ernte mit einer wirksamen Menge eines Wirkstoffes gemäss Anspruch 1 oder eines eine solche Verbindung enthaltendes Mittels behandelt.

**Claims**

1. A 5-(N-3,4,5,6-tetrahydrophthalimido)benzoic acid ester of formula I

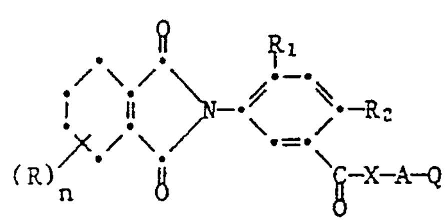

(I)

in which

- n is zero, one or two,
- R is $C_1$-$C_3$alkyl,
- $R_1$ is hydrogen or halogen,
- $R_2$ is halogen,
- X is sulfur,
- A is a straight-chain or branched $C_1$-$C_4$alkylene bridge,
- Q is a -$COOR_3$ radical,
- $R_3$ is $C_1$-$C_{20}$alkyl, $C_3$-$C_6$alkenyl, $C_3$-$C_6$alkynyl or $C_3$-$C_7$cycloalkyl,

each of which may be unsubstituted or substituted by halogen or $C_1$-$C_4$alkoxy, with the exception of the compounds
S-methoxycarbonylmethyl 2,4-dichloro-5-(3,4,5,6-tetrahydrophthalimido)-thiobenzoate,
S-ethoxycarbonyleth-1-yl 2,4-dichloro-5-(3,4,5,6-tetrahydrophthalimido)-thiobenzoate,
S-ethoxycarbonyleth-1-yl 2,4-difluoro-5-(3,4,5,6-tetrahydrophthalimido)-thiobenzoate,
S-ethoxycarbonyleth-1-yl 2-chloro-5-(3,4,5,6-tetrahydrophthalimido)-thiobenzoate,
S-ethoxycarbonyleth-1-yl 2-bromo-5-(3,4,5,6-tetrahydrophthalimido)-thiobenzoate,
S-ethoxycarbonyleth-1-yl 2-fluoro-5-(3,4,5,6-tetrahydrophthalimido)-thiobenzoate.

2. A 5-(N-3,4,5,6-tetrahydrophthalimido)benzoic acid ester according to claim 1, in which
   - n is zero, one or two,
   - R is $C_1$-$C_3$alkyl,
   - $R_1$ is hydrogen or halogen,
   - $R_2$ is halogen,
   - X is sulfur,
   - A is a straight-chain or branched $C_1$-$C_4$alkylene bridge and
   - Q is a $C_2$-$C_8$alkoxycarbonyl group.

3. S-1-Isopropoxycarbonyleth-1-yl 5-(N-3,4,5,6-tetrahydrophthalimido)-2-chloro-4-fluorothiobenzoate according to claim 1.

4. S-1-Ethoxycarbonyleth-1-yl 5-(N-3,4,5,6-tetrahydrophthalimido)-2-chloro-4-fluorothiobenzoate according to claim 1.

5. S-Decyloxycarbonylmethyl 5-(N-3,4,5,6-tetrahydrophthalimido)-2-chloro-4-fluorothiobenzoate according to claim 1.

6. S-Cyclohexyloxycarbonylmethyl 5-(N-3,4,5,6-tetrahydrophthalimido)-2-chloro-4-fluorothiobenzoate according to claim 1.

7. S-n-Butoxycarbonylmethyl 5-(N-3,4,5,6-tetrahydrophthalimido)-2-chloro-4-fluorothiobenzoate according to claim 1.

8. S-Hept-3-yloxycarbonylmethyl 5-(N-3,4,5,6-tetrahydrophthalimido)-2-chloro-4-fluorothiobenzoate according to claim 1.

9. S-Methoxyethoxycarbonylmethyl 5-(N-3,4,5,6-tetrahydrophthalimido)-2-chloro-4-fluorothiobenzoate according to claim 1.

10. S-Methoxycarbonylmethyl 5-(N-3,4,5,6-tetrahydrophthalimido)-2-chlorothiobenzoate according to claim 1.

11. S-Methoxyethoxycarbonylmethyl 5-(N-3,4,5,6-tetrahydrophthalimido)-2-chlorothiobenzoate according to claim 1.

12. S-n-Butoxycarbonylmethyl 5-(N-3,4,5,6-tetrahydrophthalimido)-2-chlorothiobenzoate according to claim 1.

13. S-Isopropoxycarbonylmethyl 5-(N-3,4,5,6-tetrahydrophthalimido)-2-chlorothiobenzoate according to

claim 1.

**14.** S-Cyclohexyloxycarbonylmethyl 5-(N-3,4,5,6-tetrahydrophthalimido)-2-chlorothiobenzoate according to claim 1.

**15.** S-Isopropoxycarbonyleth-1-yl 5-(N-3,4,5,6-tetrahydrophthalimido)-2-chlorothiobenzoate according to claim 1.

**16.** S-Methoxycarbonylmethyl 5-(N-3,4,5,6-tetrahydrophthalimido)-2-chloro-4-fluorothiobenzoate according to claim 1.

**17.** S-Isopropoxycarbonylmethyl 5-(N-3,4,5,6-tetrahydrophthalimido)-2-chloro-4-fluorothiobenzoate according to claim 1.

**18.** S-Ethoxycarbonylmethyl 5-(N-3,4,5,6-tetrahydrophthalimido)-2-chloro-4-fluorothiobenzoate according to claim 1.

**19.** S-Methoxycarbonyleth-1-yl 5-(N-3,4,5,6-tetrahydrophthalimido)-2-chlorothiobenzoate according to claim 1.

**20.** S-Methoxycarbonyleth-1-yl 5-(N-3,4,5,6-tetrahydrophthalimido)-2-chloro-4-fluorothiobenzoate according to claim 1.

**21.** S-Ethoxycarbonylmethyl 5-(N-3,4,5,6-tetrahydrophthalimido)-2-chlorothiobenzoate according to claim 1.

**22.** S-Allyloxycarbonyleth-1-yl 5-(N-3,4,5,6-tetrahydrophthalimido)-2-chloro-4-fluorothiobenzoate according to claim 1.

**23.** S-Propynyloxycarbonylmethyl 5-(N-3,4,5,6-tetrahydrophthalimido)-2-chloro-4-fluorothiobenzoate according to claim 1.

**24.** A process for the preparation of a 5-(N-3,4,5,6-tetrahydrophthalimido)benzoicacid ester of formula I according to claim 1, which process comprises reacting the anhydride of a 3,4,5,6-tetrahydrophthalic acid of formula II

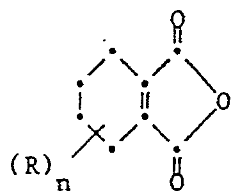

(II)

in which R and n are as defined for formula I, with the equimolar amount of a derivative of a 3-aminobenzoyl compound of formula III

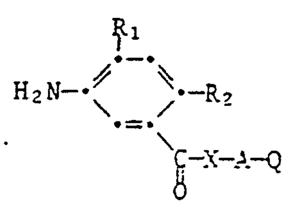

(III)

in which A, $R_1$, $R_2$, X and Q are as defined in claim 1, in an inert organic solvent or diluent and in the absence or presence of a minor amount of a dehydrating agent.

**25.** A process for the preparation of a 5-(N-3,4,5,6-tetrahydrophthalimido)-benzoic acid ester of formula I according to claim 1, which process comprises reacting a 5-(N-3,4,5,6-tetrahydrophthalimido)-benzoylhalide of formula V

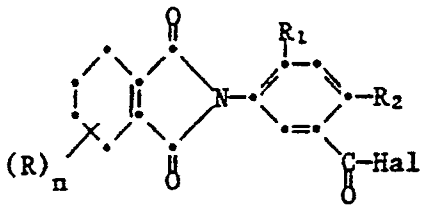

(V)

in which n, R, $R_1$ and $R_2$ are as defined for formula I and Hal is a halogen atom, preferably chlorine or bromine, with a thiol of formula VI

HX - A - Q (VI)

in which A, X and Q are as defined for formula I, in an inert organic solvent or diluent and in the presence of a base.

**26.** A process for the preparation of a 5-(N-3,4,5,6-tetrahydrophthalimido)benzoicacid ester of formula I according to claim 1, which process comprises reacting a 5-(N-3,4,5,6-tetrahydrophthalimido)-benzoicacid ester of formula VII

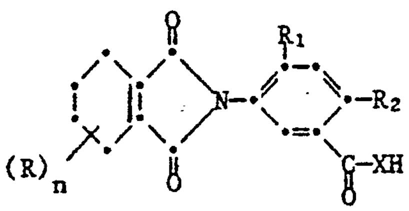

(VII)

in which n, R, $R_1$, $R_2$ and X are as defined for formula I, with an alkyl halide of formula VIII

Hal - A - Q (VIII)

in which A and Q are as defined in claim 1 and Hal is a halogen atom, preferably chlorine or bromine, in an inert organic solvent or diluent and in the presence of the molar amount of a base.

**27.** A herbicidal and plant growth regulating composition which contains, as active ingredient, a 5-(N-3,4,5,6-tetrahydrophthalimido)benzoic acid ester of formula I according to claim 1, together with carriers and/or other adjuvants.

**28.** The use of an active ingredient according to claim 1, or of a composition containing such an active ingredient, for controlling undesired plant growth.

**29.** The use of an active ingredient according to claim 1, or of a composition containing such an active ingredient, for inhibiting plant growth.

**30.** The use of an active ingredient according to claim 1, or of a composition containing such an active ingredient, for influencing plant growth in order to increase the yield.

**31.** A pre- or postemergence method of selectively controlling weeds in crops of useful plants, which method comprises treating said crops of useful plants or the crop area thereof with an effective amount of an active ingredient of formula I according to claim 1, or of a composition containing such a compound.

32. A method of suppressing plant growth beyond the 2-leaf stage, which method comprises treating the plants during their growth with an effective amount of an active ingredient of formula I according to claim 1, or of a composition containing such an active ingredient.

33. A method of defoliating and desiccating crops of cotton and potatoes in order to facilitate the harvest thereof, which method comprises treating the crop, shortly before harvesting, with an effective amount of an active ingredient according to claim 1, or of a composition containing such a compound.

**Revendications**

1. Esters 5-(N-3,4,5,6-tétrahydrophtalimido)-benzoïques de formule I

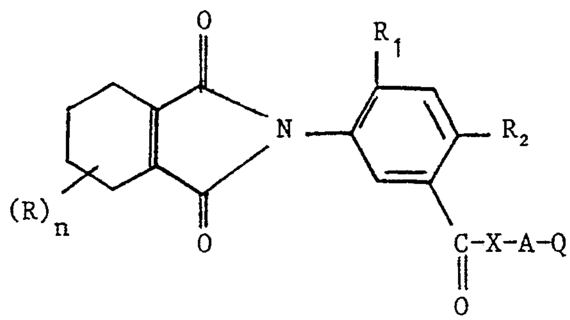

(I)

dans laquelle

- n est égal à 0, 1 ou 2,
- R représente un groupe alkyle en C 1-C 3,
- $R_1$ représente l'hydrogène ou un halogène,
- $R_2$ représente un halogène,
- X représente le soufre,
- A représente un pont alkylène à chaîne droite ou ramifiée en C 1-C 4,
- $R_3$ représente un groupe alkyle en C 1-C 20, alcényle en C 3-C 6, alcynyle en C 3-C 6 ou cycloalkyle en C 3-C 7, non substitué ou qui peut être substitué par des halogènes ou des groupes alcoxy en C 1-C 4,

à l'exception des composés suivants :
2,4-dichloro-5-(3,4,5,6-tétrahydrophtalimido)-thiobenzoate de S-méthoxycarbonylméthyle,
2,4-dichloro-5-(3,4,5,6-tétrahydrophtalimido)-thiobenzoate de S-éthoxycarbonyléth-1-yle,
2,4-difluoro-5-(3,4,5,6-tétrahydrophtalimido)-thiobenzoate de S-éthoxycarbonyléth-1-yle,
2-chloro-5-(3,4,5,6-tétrahydrophtalimido)-thiobenzoate de S-éthoxycarbonyléth-1-yle,
2-bromo-5-(3,4,5,6-tétrahydrophtalimido)-thiobenzoate de S-éthoxycarhonyléth-1-yle,
2-fluoro-5-(3,4,5,6-tétrahydrophtalimido)-thiobenzoate de S-éthoxycarbonyléth-1-yle.

2. Esters 5-(N-3,4,5,6-tétrahydrobenzimido)-benzoïques selon revendication 1, pour lesquels

- n est égal à 0, 1 ou 2,
- R représente un groupe alkyle en C 1-C 3,
- $R_1$ représente l'hydrogène ou un halogène,
- $R_2$ représente un halogène,
- X représente le soufre,
- A représente un pont alkylène à chaîne droite ou ramifiée en C 1-C 4, et
- Q représente un groupe alcoxycarbonyle en C 2-C 8.

3. Le thioester 1-isopropoxycarbonyléth-1-ylique de l'acide 5-(N-3,4,5,6-tétrahydrophtalimido)-2-chloro-4-fluoro-benzoïque, selon revendication 1.

4. Le thioester 1-éthoxycarbonyléth-1-ylique de l'acide 5-(N-3,4,5,6-tétrahydrophtalimido)-2-chloro-4-fluoro-benzoïque, selon revendication 1.

5. Le thioester décyloxycarbonylméthylique de l'acide 5-(N-3,4,5,6-tétrahydrophtalmido)-2-chloro-4-fluoro-benzoïque selon revendication 1.

6. Le thioester cyclohexyloxycarbonylméthylique de l'acide 5-(N-3,4,5,6-tétrahydrophtalimido)-2-chloro-4-fluoro-benzoïque, selon revendication 1.

7. Le thioester n-butoxycarbonylméthylique de l'acide 5-(N-3,4,5,6-tétrahydrophtalimido)-2-chloro-4-fluoro-benzoïque, selon revendication 1.

8. Le thioester hept-3-yloxycarbonylméthylique de l'acide 5-(N-3,4,5,6-tétrahydrophtalimido)-2-chloro-4-fluoro-benzoïque, selon revendication 1.

9. Le thioester méthoxyéthoxycarbonylméthylique de l'acide 5-(N-3,4,5,6-tétrahydrophtalimido)-2-chloro-4-fluoro-benzoïque, selon revendication 1.

10. Le thioester méthoxycarbonylméthylique de l'acide 5-(N-3,4,5,6-tétrahydrophtalimido)-2-chloro-benzoïque, selon revendication 1.

11. Le thioester méthoxyéthoxycarbonylméthylique de l'acide 5-(N-3,4,5,6-tétrahydrophtalimido)-2-chloro-benzoïque, selon revendication 1.

12. Le thioester n-butoxycarbonylméthylique de l'acide 5-(N-3,4,5,6-tétrahydrophtalimido)-2-chloro-benzoïque, selon revendication 1.

13. Le thioester isopropoxycarbonylméthylique de l'acide 5-(N-3,4,5,6-tétrahydrophtalimido)-2-chloro-benzoïque, selon revendication 1.

14. Le thioester cyclohexyloxycarbonylméthylique de l'acide 5-(N-3,4,5,6-tétrahydrophtalimido)-2-chloro-benzoïque, selon revendication 1.

15. Le thioester isopropoxycarbonyléth-1-ylique de l'acide 5-(N-3,4,5,6-tétrahydrophtalimido)-2-chloro-benzoïque, selon revendication 1.

16. Le thioester méthoxycarbonylméthylique de l'acide 5-(N-3,4,5,6-tétrahydrophtalimido)-2-chloro-4-fluoro-benzoïque, selon revendication 1.

17. Le thioester isopropoxycarbonylméthylique de l'acide 5-(N-3,4,5,6-tétrahydrophtalimido)-2-chloro-4-fluoro-benzoïque, selon revendication 1.

18. Le thioester éthoxycarbonylméthylique de l'acide 5-(N-3,4,5,6-tétrahydrophtalimido)-2-chloro-4-fluoro-benzoïque, selon revendication 1.

19. Le thioester méthoxycarbonyléth-1-ylique de l'acide 5-(N-3,4,5,6-tétrahydrophtalimido)-2-chloro-benzoïque, selon revendication 1.

20. Le thioester méthoxycarbonyléth-1-ylique de l'acide 5-(N-3,4,5,6-tétrahydrophtalimido)-2-chloro-4-fluoro-benzoïque, selon revendication 1.

21. Le thioester éthoxycarbonylméthylique de l'acide 5-(N-3,4,5,6-tétrahydrophtalimido)-2-chloro-benzoïque, selon revendication 1.

22. Le thioester allyloxycarbonyléth-1-ylique de l'acide 5-(N-3,4,5,6-tétrahydrophtalimido)-2-chloro-4-fluoro-benzoïque, selon revendication 1.

23. Le thioester propynyloxycarbonylméthylique de l'acide 5-(N-3,4,5,6-tétrahydrophtalimido)-2-chloro-4-fluoro-benzoïque, selon revendication 1.

24. Procédé de préparation des esters 5-(N-3,4,5,6-tétrahydrophtalimido)-benzoïques de formule I, revendication 1, caractérisé en ce que l'on fait réagir l'anhydride d'un acide 3,4,5,6-tétrahydrophtalique de formule II

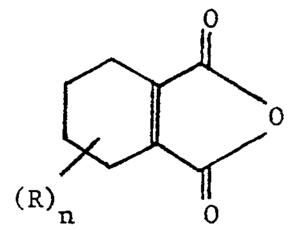

(II)

dans laquelle R et n ont les significations indiquées en référence à la formule I, dans un solvant ou diluant organique inerte, éventuellement en présence d'une petite quantité d'un agent déshydratant, avec la quantité équimoléculaire d'un dérivé 3-aminobenzoylique de formule III

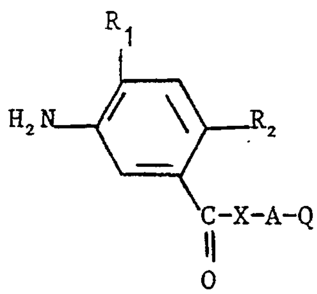

(III)

dans laquelle A, $R_1$, $R_2$, X et Q ont les significations indiquées en référence à la formule I.

**25.** Procédé de préparation des esters 5-(N-3,4,5,6-tétrahydrophtalimido)-benzoïques de formule I, revendication 1, caractérisé en ce que l'on fait réagir un halogénure d'acide 5-(N-3,4,5,6-tétrahydrophtalimido)-benzoïque de formule V

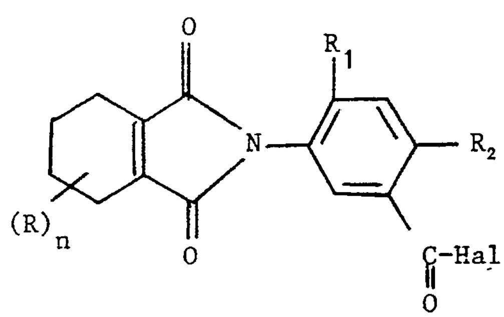

(V)

dans laquelle n, R, $R_1$ et $R_2$ ont les significations indiquées en référence à la formule I et Hal représente un atome d'halogène, de préférence de chlore ou de brome, dans un solvant ou diluant organique inerte, en présence d'une base, avec un thiol de formule VI

HX - A - Q (VI)

dans laquelle A, X et Q ont les significations indiquées en référence à la formule I.

**26.** Procédé de préparation des esters 5-(N-3,4,5,6-tétrahydrophtalimido)-benzoïques de formule I, revendication 1, caractérisé en ce que l'on fait réagir un acide 5-(N-3,4,5,6-tétrahydrophtalimido)-benzoïque de formule VII

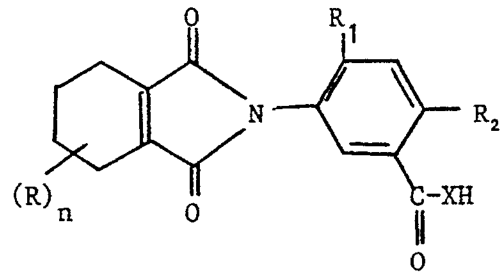

(VII)

dans laquelle n, R, $R_1$, $R_2$ et X ont les significations indiquées en référence à la formule I, dans un solvant ou diluant organique inerte, en présence de la quantité molaire d'une base, avec un halogénure d'alkyle de formule VIII

Hal - A - Q (VIII)

dans laquelle A et Q ont les significations indiquées dans la revendication 1, et Hal représente un atome d'halogène, de préférence de chlore ou de brome.

**27.** Un produit herbicide et régulateur de la croissance des végétaux, caractérisé en ce qu'il contient en tant que substance active, avec des véhicules et/ou d'autres additifs, un ester 5-(N-3,4,5,6-tétrahydrophtalimido)-benzoïque de formule I, revendication 1.

**28.** L'utilisation d'une substance active selon revendication 1 ou d'un produit contenant une telle substance active pour la lutte contre les croissances de végétaux indésirables.

**29.** L'utilisation d'une substance active selon revendication 1 ou d'un produit contenant une telle substance active pour l'inhibition de la croissance des végétaux.

**30.** L'utilisation d'une substance active selon revendication 1 ou d'un produit contenant une telle substance active pour agir sur la croissance des végétaux en vue d'une augmentation des rendements.

**31.** Procédé pour combattre sélectivement en pré-levée ou en post-levée les végétaux adventices dans les cultures de végétaux utiles, caractérisé en ce que l'on traite les cultures de végétaux utiles ou leur aire de culture par une quantité efficace d'une substance active de formule I, revendication 1, ou d'un produit contenant un tel composé.

**32.** Procédé pour inhiber la croissance des végétaux au-delà du stade deux feuilles, caractérisé en ce que l'on traite les végétaux en cours de croissance par une quantité efficace d'une substance active de formule I, revendication 1, ou d'un produit contenant une telle substance active.

**33.** Procédé pour la défoliation et la dessiccation des cultures de coton et de pommes de terre en vue de faciliter la récolte, caractérisé en ce que l'on traite la culture, peu avant la récolte, par une quantité efficace d'une substance active selon revendication 1 ou d'un produit contenant un tel composé.